# EUROPEAN PATENT APPLICATION

(11) **EP 1 676 635 A2**
(43) Date of publication of application: **05.07.2006**
(21) Application number: 06004827.9
(22) Date of filing: 30.07.2003
(51) Int. Cl.: B01J 31/18, C07C 29/145, C07C 209/42, C07C 211/27, C07C 217/64, C07C 247/10, C07B 37/04, C07B 37/12, C07B 53/00, C07C 5/02, C07C 247/06, C07C 251/02, C07D 203/06, C07D 301/03, C07D 301/14, C07D 301/32

(54) **Immobilisable ligands derived from ethylenediamine**

(30) Priority: 12.08.2002 GB 0218675
(62) Divisional of application: 03784236.6
(71) Applicant: Johnson Matthey PLC, 40-42 Hatton Garden London EC1N 8EE (GB)
(72) Inventor: Hems, William, Norwich Norfolk NR14 7RG (GB); Xiao, Jianliang, Liverpool Merseyside L16 3GJ (GB); Chen, Weiping, Liverpool Merseyside L16 3GB (GB)
(74) Representative: Ridland, John

(57) **Abstract**

A ligand of formula (I) is described 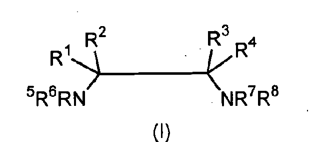 wherein R¹, R³, R⁵, R⁶, R⁷ and R⁸ are hydrogen, characterized in that R² and R⁴ are functional group-containing aryl groups in which the functional groups are selected from the group consisting of halogen (Cl, Br, F or I), hydroxyl, alkoxy, carbonyl, carboxyl, anhydride, carbene, methacryl, epoxide, vinyl, nitrile, mercapto, amine, imine, amide and imide. The ligand is useful for preparing immobilised catalysts for performing e.g. asymmetric catalysis.

## Description

This invention relates to nitrogen-containing ligands and in particular to nitrogen-containing ligands supported on polymers, metal oxides or silica materials that provide a means for immobilising metal catalysts. Such immobilised metal catalysts are useful for accelerating and directing chemical reactions whose products are useful, for example, as chemical intermediates or reagents for use in the production of fine chemicals or pharmaceutical intermediates.

Nitrogen-containing ligands are a particularly useful group of ligands which find widespread use in asymmetric catalysis (see "Nitrogen-containing ligands for asymmetric homogeneous and heterogeneous catalysis", Fache et al, *Chem. Rev.,* 2000, 100, 2159-2231).

The fixing of homogeneous catalysts to solid supports provides the potential for extending the benefits of heterogeneous catalysts to homogeneous systems. These benefits include easier separation of catalyst and reaction products leading to shorter work up times and improved process efficiency, the potential for re-activation and re-use of the supported catalysts which are often based on expensive metals and complex ligand geometry, and the possible adaptation of the immobilised catalyst to continuous flow fixed-bed processes.

Strategies for homogeneous catalyst immobilisation have been based on absorption, ion exchange or tethering the catalysts to a support using covalent attachment. By covalent attachment we mean the formation of a covalent bond between support and catalyst. Covalent attachment is attractive for providing catalysts that may be more robust to catalyst leaching and hence retain higher activities upon re-use. Covalent attachment of the metal catalyst may be achieved by forming chemical bonds between a ligand and particles of a polymer, for example polystyrene, or oxide material, for example silica, that has been subjected to a surface functionalisation

Immobilisation of nitrogen-containing ligands, such as diamines to polymer supports is known, but attempts at covalent immobilisation of such ligands has been restricted to formation of bonds via functional groups linked to the nitrogen atom(s) of the ligands. For example the amine group may be reacted with a benzenesulphonyl-functionalised polymer (see Lemaire et al, *Synlett.,* 1997, 1257 and Williams et al, *Tetrahedron Lett*., 2001, 42, 4037) or alternatively the amine group may be reacted with chlorosulphonylbenzoic acid and the resulting functionalised diamine reacted with aminomethylated polystyrene. (see Bayston et al, *Tetrahedron. Asymm.,* 1998, 9, 2015). These 'nitrogen- immobilised' diamines are depicted below.

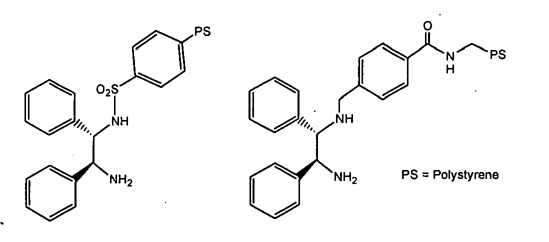

These catalysts however do not provide high levels of activity or selectivity, particularly upon re-use. For example, the ruthenium-catalysed reduction of acetophenone using the latter ligand was examined and the activity of the catalyst was found to decline markedly upon reuse and be dependent on the type of polymer used. It therefore appears that the immobilisation of nitrogen-containing ligands via the nitrogen atom(s) is undesirable because the presence of the linking groups on the nitrogen atoms effects the stability, activity and/or the selectivity of the catalysts.

Furthermore, the presence of a linking group attached to the nitrogen atom(s) of such nitrogen-containing ligands prevents useful derivatives such as Schiff bases or cationic ligands from being readily prepared.

We have found that immobilisation of the nitrogen-containing ligands may advantageously be performed via functional groups attached to carbon atoms linking the nitrogen atoms.

It has been proposed in PCT application WO 02/066159 to prepare an immobilised ligand by reaction of a functional group-containing ligand with an organofunctional silica, wherein the organofunctional silica is prepared by co-hydrolysis of an alkyl silicate and an organofunctional silane. That application specifically discloses immobilised phosphine ligands. While the aforementioned PCT application also suggested that nitrogen containing ligands such as diamines and Schiff bases could be employed as a functional group-containing ligands, it did not specifically disclose such ligands having functional groups attached to the carbon atoms linking the nitrogen atoms.

Accordingly the present invention provides an immobilised nitrogen-containing ligand comprising the reaction product of a compound of formula (I) 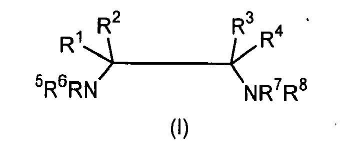 wherein R⁵, R⁶, R⁷ and R⁸ are independently hydrogen, a saturated or unsaturated C1-C10 alkyl group, an aryl group, a urethane group, a sulphonyl group or form an imine group, R¹, R², R³ and R⁴ are independently hydrogen, a saturated or unsaturated C1-C10 alkyl group or an aryl group and at least one of R¹, R², R³ and R⁴ is functionalised with a functional group, and a solid support having a site capable of reacting with said functional group.

Preferably R¹ and R³ are hydrogen and at least one of R² and R⁴ is a functional group-containing aryl group. Where R⁵, R⁶, R⁷ and R⁸ are saturated or unsaturated C1-C10 alkyl groups or aryl groups, it will be understood that these groups may themselves further comprise functional groups selected from the list comprising halogen (Cl, Br, F or I), hydroxyl, carbonyl, carboxyl, nitrile, mercapto, alkoxy, amine, imine, amide and imide. Thus, in one embodiment the nitrogen containing ligand is a diamine where preferably at least one of R⁵, R⁶, R⁷ and R⁸ are hydrogen, more preferably all of R⁵, R⁶, R⁷ and R⁸ are hydrogen. In a further embodiment, NR⁵R⁶ and NR⁷R⁸ form imine (N=C) groups whereby R⁶ and R⁸ are omitted and R⁵ and R⁷ are as defined above. Examples of these embodiments, where R¹ and R³ are hydrogen and R² and R⁴ are functional group-containing aryl groups are depicted below;

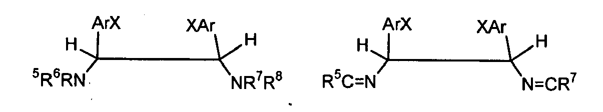

Preferably R¹, R², R³ and R⁴ are chosen such that the nitrogen atoms are bonded to chiral centres and the nitrogen-containing ligand is chiral. The ligand may be homochiral, i.e. (R,R) or (S,S) or have one (R) and one (S) centre. Preferably the ligand is homochiral.

The functional group on at least one of R¹, R², R³ and R⁴ that may be used to bond to the support may be any that is capable of reacting with the support material and which does not prevent the catalytically active metal complex from reacting with the ligand. One or more functional groups may be present which may be the same or different. Such groups include halogen (Cl, Br, F or I), hydroxyl, alkoxy, carbonyl, carboxyl, ,anhydride, carbene, methacryl, epoxide, vinyl, nitrile, mercapto, amine, imine, amide and imide. The functional groups to be reacted with the solid support may conveniently be introduced into the nitrogen containing ligand during its preparation.

Suitable nitrogen containing ligands include but are not restricted to the following where X and Y are functional groups as defined above

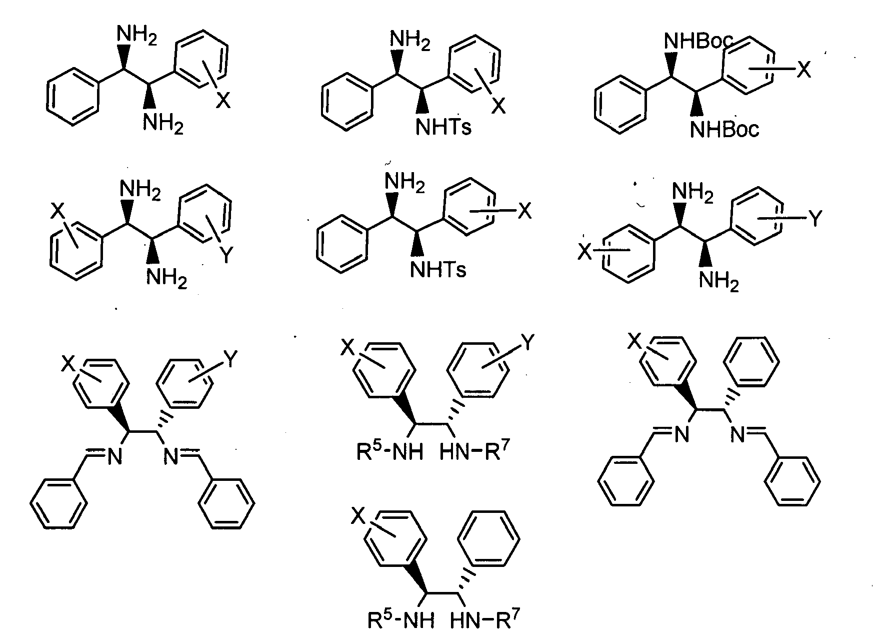

If a functional group available on the nitrogen-containing ligand is unsuitable for reaction with the solid support, it may be converted by chemical reaction or alternatively, the ligand may be reacted with a linker molecule that provides a suitable functional group capable of reaction with the solid support. The linker molecule should therefore contain functional groups capable of reacting with at least one of R¹, R², R³ and R⁴ and the solid support.

Accordingly the invention further provides a linker-modified nitrogen-containing ligand of formula (I) 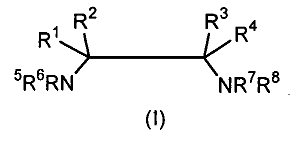 wherein R⁵, R⁶, R⁷ and R⁸ are independently hydrogen, a saturated or unsaturated C1-C10 alkyl group, an aryl group, a urethane group, a sulphonyl group or form an imine group, R¹, R², R³ and R⁴ are independently hydrogen, a saturated or unsaturated C1-C10 alkyl group or an aryl group and at least one of R¹, R², R³ and R⁴ is a aryl group substituted with a linking compound selected from C1-C10 alkyl, alkoxy, alkyl-aryl, aryl, phenoxy or anilide compounds containing functional groups selected from halide, hydroxyl, carbonyl, carboxyl, anhydride, carbene, methacryl, epoxide, vinyl, nitrile, mercapto, isocyanate, amine, imine, amide and imide.

In a preferred embodiment R¹, R³, R⁵, R⁶, R⁷ and R⁸ are hydrogen and the linker molecule is a polyethylene glycol. More preferably the linker molecule is PEG 2000 (i.e. a PEG having a molecular weight of about 2000). Advantages of preparing the nitrogen-containing ligand in this way are that new functional groups may be introduced in a way that is not generally possible in commercially available starting materials and that functional groups may be introduced at a greater distance from the nitrogen atom.

Methods for preparing enantiomerically pure, non-functionalised 1,2-Nitrogen-containing ligands such as 1,2-diamines, either directly by asymmetric synthesis or by synthesis of racemic mixtures followed by chiral resolution are known. We have found however that many of the routes to non-functionalised ligands do not work well for functionalised ligands. We therefore have developed a new route to preparing functionalised 1,2-diamines and their derivatives such as Schiff bases or cationic compounds. Whereas in the presence of 0.1 mol% of RuCl[(R,R)-Tsdpen)(η⁶-p-cymene), using a mixture of HCOOH-Et₃N (3.1:2.6) as hydrogen source, asymmetric transfer hydrogenation of benzils affords the (S,S)-hydrobenzoins quantitatively with high diastereomeric (>95% de) and enantiomeric purities (>99% ee), we have found that under the same conditions, replacement of the benzils with functionalised benzoins gives functionalised (S,S)-hydrobenzoins with same yield, de and ee, in which the benzoin with a chirally labile stereogenic center is converted to one major stereoisomer, (S,S)-product, via dynamic kinetic resolution. As functionalised benzoins can be readily prepared from the corresponding functionalised benzaldehydes by benzoin condensation, this route provides an efficient method to prepare enantiomerically pure functionalised 1,2-diarylethylenediamines. This route is depicted below;

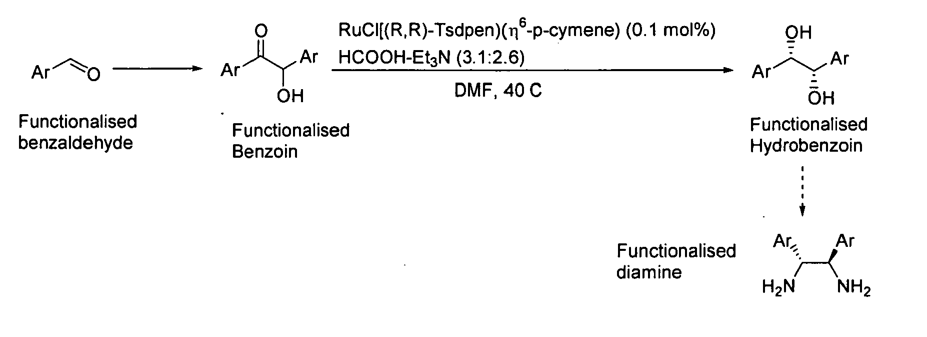

Thus the invention further provides a method for preparing an immobilised ligand of formula (I) wherein R¹ and R³ are hydrogen, R² and R⁴ are functional group-containing aryl groups and R⁵, R⁶, R⁷ and R⁸ are hydrogen, 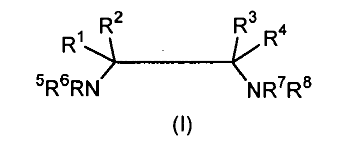 comprising the steps of;
(a) Performing a benzoin condensation on a functionalised benzaldehyde to give a functionalised benzoin,
(b) reducing the functionalised benzoin to give a functionalised hydrobenzoin,
(c) transforming the functionalised hydrobenzoin into a functionalised 1,2-diarylamine, and
(d) reacting the functionalised 1,2-diarylamine with a solid support having a site capable of reacting with said functionalised 1,2-diarylamine to form an immobilised ligand.

The functional group on the aryl group may be in the ortho, meta or para position. However, when the substituent is at the meta-position of the phenyl ring it minimizes the electronic effects on the amino group which may facilitate the synthesis of the diamine. Thus in a preferred embodiment the functionalised diamine is an enantiomerically pure 1,2-di-(*meta*-substituted phenyl)ethylenediamine. The synthesis of a 1,2-di-(*meta*-substituted phenyl)ethylenediamine begins, for example, with the *meta*-substituted benzaldehyde. By benzoin condensation, the corresponding benzoins, are obtained in over 90% yield. Reduction of the functionalised benzoins may be performed by an asymmetric hydrogen transfer hydrogenation using a chiral Ru catalyst. For example, in the presence of 0.1 mol% of RuCl[(R,R)-Tsdpen)(η⁶-p-cymene), using a mixture of HCOOH-Et₃N (3.1:2.6) as hydrogen source, asymmetric transfer hydrogenation of the substituted benzoins affords the substituted (S,S)-hydrobenzoins, quantitatively with high diastereomeric (>95% de). Conversion of the functionalised hydroxy benzoin to the functionalised 1,2-diarylamine may be performed by (a) forming the diazide, and (b) converting the diazide into the diamine. This is preferably performed using the tosyl derivative of the functionalised hydroxybenzoin. Treatment of the (S,S)-hydrobenzoin with tosyl chloride (TsCl) in pyridine gives the corresponding 1,2-ditosyloxy derivatives, which may be used directly for next step without purification. By reacting the 1,2-ditosyloxy derivatives with sodium azide in DMF at 75-80°C for 8 h, the (R,R)-1,2-diazides, are obtained in good yield. Finally, the (R,R)-1,2-diazides may be reduced to substituted 1,2-(R,R)-diphenylethylenediamines in almost quantitative yield by LiAlH₄ in Et₂O.

As stated above, linker groups may be reacted with the ligands prior to reaction of the linker-modified ligand with the solid support. For example, 1,2-(R,R)-diphenylethylenediamines may be reacted with linking groups such a polyethylene glycol (PEG) to prepare soluble PEG modified 1,2-(R,R)-diphenylethylenediamines. This may be performed for example by protection of the benzyl-functionalised DPEN or TsDPEN with Boc, hydrogenolysis, reaction with polyethylene glycol, e.g. PEG 2000 as the monomethyl ether mesylate and removal of Boc. The polyethylene glycol monomethyl ether mesylate may be prepared from polyethylene glycol, e.g. PEG 2000 monomethyl ether by sulfonation with mesylclhoride (MsCl) in the presence of base. To regenerate the PEG hydroxyl groups, the methyl ether may be e.g. hydrolysed using known methods.

It is possible to inter-convert the diamines of the present invention using known methods to provide the imine and cationic ligands. For example, reaction of the amine groups with aldehydes or ketones provides imines. An example of this reaction is depicted below; 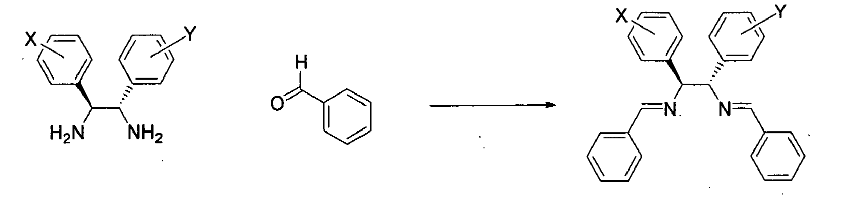 The inter-conversion reactions may be performed upon the diamine ligand before or after reaction with the solid support.

The solid support materials to which the nitrogen-containing ligand having a functional group is covalently bonded, may be polymers, metal oxides or organofunctional silica materials that have sites capable of reacting with said functional group. The metal oxides include silica, titania, zirconia or alumina, or mixtures of these. The polymers may be any that are insoluble in the solvent system used for performing the catalysed reaction and are stable under the reaction conditions. Preferably, where the reaction is performed in polar solvents, the polymer is a polystyrene or a polystyrene copolymer of suitable molecular weight, which may be further functionalised, e.g. with polyethyleneglycol or aminomethyl groups. By the term "organofunctional silica materials" we mean organofunctional silica materials prepared, for example, by hydrolysis of organofunctional silanes, preferably in the presence of alkyl silicates and optionally other metal alkoxides.

The solid support materials have reactive sites capable of reaction with the functional group-containing ligand. These reactive sites may be selected from halide (Cl, Br, F, or I), hydroxyl, carbonyl, carboxyl, anhydride, carbene, methacryl, epoxide, vinyl, nitrile, mercapto, isocyanate, amine, imine, amide and imide.

In the case of metal oxides, the sites on the solid support that react with the functional group-containing ligand may be surface hydroxyl groups, which are present on the hydrated surfaces, or may be the result of surface functionalisation. Surface functionalisation of the oxides may be carried our using a variety of organic compounds such as carboxylic acids, anhydrides, phosphates, or sulphonates or metal-organic compounds such as organic titanates, aluminates, zirconates or organofunctional silanes. Preferably surface functionalisation is performed using organofunctional silanes. Organofunctional silanes useful for the present invention include vinyltrimethoxy silane, vinyltriethoxysilane, dichlorodivinylsilane, 3-(aminopropyl)trimethoxysilane, 3-(aminopropyl)triethoxysilane, [3-(methacryloyloxy)-propyl]trimethoxysilane, [3-(tri(ethoxy/methoxy)silyl]propyl]urea, 3-(glycidoxypropyl)trimethoxysilane, 4-(triethoxysilyl)butyronitrile, 3-(Iodopropyl)trimethoxysilane, 3-(mercaptopropyl)-trimethoxysilane, 3-(triethoxysilyl)propionitrile, 4-(triethoxysilyl)butyronitrile , ((chloromethyl)-phenylethyl)trimethoxysilane, and ((chloromethyl)phenyl)trimethoxysilane and mixtures of these.

Polymers often may be prepared by addition or condensation reactions of suitable monomers that include at least a proportion of a monomer having a suitable reactive site capable of reaction with the functional group-containing ligand. Alternatively a reactive site may be grafted on or formed by a functional group inter-conversion reaction. By functional group inter-conversion we mean changing the reactive site on the polymer to enable reaction with the functional group-containing ligand. For example, if the functional group containing-ligand has a pendant hydroxyl group capable of reaction with the polymer having a reactive site, it may be desirable to chemically convert the reactive site on the polymer from, for example a cyano-group to a carboxyl group or, a carboxyl group to an acid-chloride group, to facilitate a reaction between said polymer and the ligand. Furthermore the polymer may have one or more reactive sites. The advantage of this feature is that the support may be tailored to react with different ligands.

The polymers should be insoluble and stable under the reaction conditions. Polystyrene polymers and polystyrene functionalised with PEG of suitable molecular weight are preferred. A polystyrene functionalised with PEG, having a bromine-atom reactive site is available commercially as Tentagel S-Br. For example a polymer-supported 1,2-(R,R)-diphenylethylenediamine may be prepared by reaction of a hydroxyl-functionalised 1,2-(R,R)-diphenylethylenediamine.

The organofunctional silica materials are preferably those as described in the aforementioned PCT application WO 02/066159, herein incorporated by reference, prepared by the co-hydrolysis of an alkyl silicate and an organofunctional silane.

A reaction of a diamine containing a functional group with an organofunctional silica to provide a supported diamine is depicted below. Here Y represents the functional group on the diamine capable of reaction with the organofunctional silica and A represents the group resulting from the reaction of X and Y that bonds the diamine to the organofunctional silica. In an alternative embodiment, one of the diamine nitrogen atoms is protected with a removable group, e.g. a tosyl group;

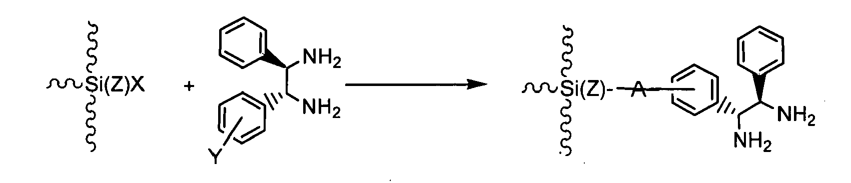

The reaction may be achieved by any effective chemical reaction between the functional groups of the organofunctional silica and the nitrogen-containing ligand. Typical reactions include for example, esterification reactions, amidation reactions, addition reactions, substitution reactions, insertion reactions and carbon-carbon coupling reactions and may be performed by any method known to those skilled in the art. For example, esterification reactions may be performed between diamine and silica either having carboxyl and hydroxyl groups, anhydride and hydroxyl groups or acid-chloride and hydroxyl groups in the presence of suitable catalysts or reagents. Amidation reactions may be performed between ligand and silica either having carboxyl groups and primary or secondary amine groups or anhydride groups and primary or secondary amine, again in the presence of suitable catalysts or reagents.

In order to prepare an immobilised catalyst, a metal compound is reacted with the immobilised nitrogen-containing ligand.

Accordingly the invention further provides an immobilised catalyst comprising the reaction product of an immobilised nitrogen-containing ligand of formula (I) 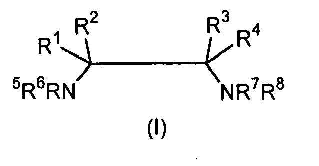 wherein R⁵, R⁶, R⁷ and R⁸ are independently hydrogen, a saturated or unsaturated C1-C10 alkyl group, an aryl group, a urethane group, a sulphonyl group or form an imine group, R¹, R², R³ and R⁴ are independently hydrogen, a saturated or unsaturated C1-C10 alkyl group or an aryl group and at least one of R¹, R², R³ and R⁴ is bound to a solid support, and a metal compound.

The metal compound is preferably a compound of Sc, Zr, Hf, Nb, Ta, Cr, Mo, W, Mn, Tc, Re, Fe, Ru, Co, Ni, Pd, Pt, Cu, Ag, Al, Ge, Sb or Sn. Preferably, for asymmetric hydrogenation reactions, the metal compound is a compound of Pd, Pt or Ru, particularly Ru; preferably for hydrolytic kinetic resolution of epoxides the metal compound is a Co compound; preferably for ring-opening reactions the metal compound is a Cr or an Al compound; and preferably for Heck reactions the metal compound is a Pd compound. The metal compound may be elemental metal, but is preferably a metal salt, e.g. halide, carboxylate, sulphonate or phosphonate, a metal alkyl or organometallic compound. For example rhodium may be reacted as a 1,5-cyclooctadiene complex and for manganese, palladium or cobalt the metal may be reacted as the di-acetate. Particularly suitable metal compounds for preparing hydrogenation catalysts include [RuCl₂(benzene)]₂ and [RuCl₂(cymene)]₂ that may additionally be reacted with a chiral phosphine. Preferably the chiral phosphine is a chiral bis(phosphine). A range of chiral bis(phosphines) are known and may be used in the present invention. Suitable chiral bis(phosphines) include but are not restricted to BINAP, Tol-BINAP, MeO-BIPHEP, DUPHOS, PHANEPHOS, DPPF and P-PHOS.

Reactions of a metal compound with a supported diamine to provide (a) a polymer-supported catalyst, and (b) an organofunctional silica material-supported catalyst are depicted below.

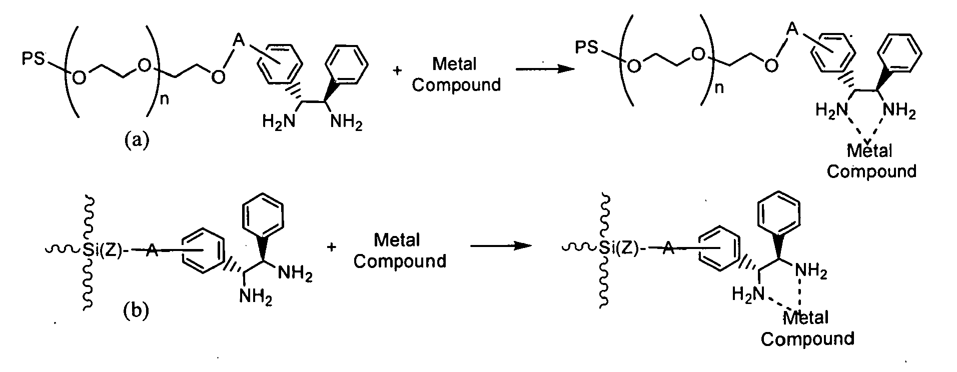

The reaction between metal compound and immobilised nitrogen-containing ligand may be achieved by methods known to those skilled in the art and is preferably effected by reaction of a metal compound with the nitrogen-containing ligand in a suitable solvent. Such reactions include, for example, ligand substitution reactions and metal-insertion reactions. The metal may also, if desired, be subjected to steps of oxidation or reduction to provide the necessary catalytic activity. For example cobalt catalysts may be oxidised from Co(II) to Co(III).

As stated above, preferably the supported nitrogen-containing ligands of the present invention are chiral ligands providing supported chiral catalysts. The supported chiral catalysts of the present invention may be applied to a large number of asymmetric reactions used to produce chiral products. Such reactions include hydrogenation reactions, including transfer hydrogenation reactions, dihydroxylation reactions, hydrolysis reactions, carbon-carbon bond formation reactions such as Heck or Suzuki reactions, hydroamination reactions, epoxidations, Aldol reactions, aziridinations, cycloadditions, hetero-Diels-Alder reactions, hetero-ene reactions, Claisen rearrangements, carbonyl reductions, sigmatropic rearrangements, additions of nucleophiles to π-bonds, addition of nucleophiles to carbonyl groups and ring-opening reactions. Preferably the reactions are hydrogenation reactions, transfer hydrogenation reactions, hydrolysis reactions and carbon-carbon bond formation reactions. The advantages of the catalysts of the present invention are that they are readily separated from the reaction products and may be re-used if so desired.

The invention is illustrated by the following examples.

### Example 1: Preparation of Diamine ligands

Functionalised 1,2-diarylamines were prepared according to the following scheme; 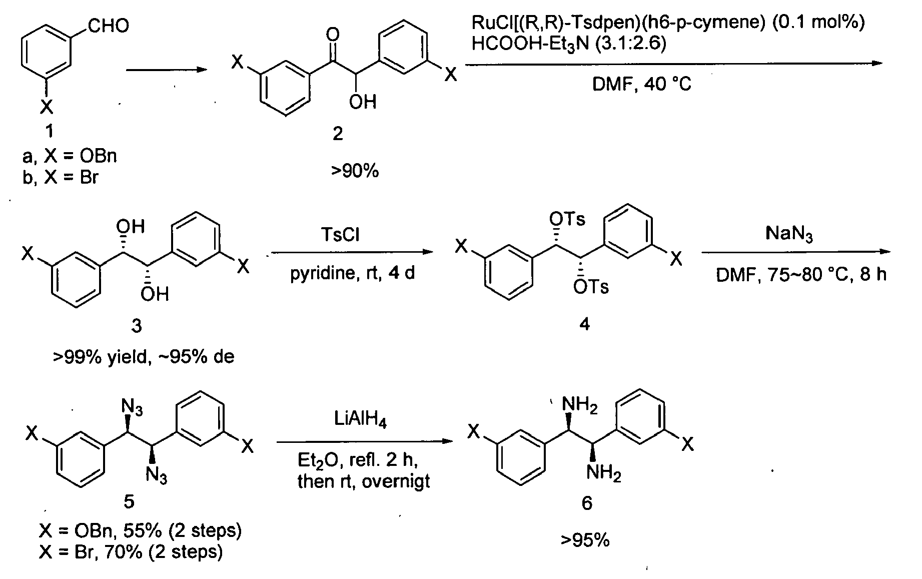
(a) Preparation of 3-Benzyloxybenzaldehyde (1a):
   A suspension of 3-hydroxybenzaldehyde (36.64 g, 300 mmol), benzyl chloride (41.77 g, 330 mmol), anhydrous K₂CO₃ (49.76 g, 360 mmol) and Nal (1.0 g) in absolute EtOH (400 ml) was stirred and refluxed for 15 h. After cooling to room temperature, the salts were filtered off, and washed with CH₂Cl₂ (100 ml). The filtrate was evaporated under reduced pressure. The residue was dissolved in EtOAc (300 ml), washed with water (100 ml) and brine (100 ml), dried (MgSO₄), and evaporated under reduce pressure. The residue was recrystallized from MeOH to give the product (63.67 g, 96%) as colorless crystals. ¹H NMR (CDCl3, δ): 9.97 (s, 1H), 7.22~7.48 (m, 9H), 5.12 (s, 2H).
(b) Preparation of 3,3'-Dibenzyloxybenzoin (2a):
   A solution of 3-benzyloxybenzaldehyde (21.23 g, 100 mmol) and NaCN (2.50 g) in EtOH (100 ml) and water (40 ml) was refluxed for 8 h. Most of EtOH was removed under reduced pressure. The residue was dissolved in EtOAc (200 ml), washed with water (2x100 ml) and brine (100 ml), dried (MgSO₄), and evaporated under reduced pressure. The residue was recrystallized from MeOH to give the product (19.52 g, 92%) as colorless crystals. ¹H NMR (CDCl₃, δ): 6.87~ 7.53 (m, 18 H), 5.84 (d, J = 3.8 Hz, 1 H), 5.04 (s, 2H), 5.00 (s, 2H), 4.51 (d, J = 3.8 Hz, 1H).
(c) Preparation of 3,3'-Dibromobenzoin (2b):
   A solution of 3-bromobenzaldehyde (18.50 g, 100 mmol) and NaCN (2.50 g) in EtOH (50 ml) and water (20 ml) was refluxed for 8 h. Most of EtOH was removed under reduced pressure. The residue was dissolved in EtOAc (200 ml), washed with water (2x100 ml) and brine (100 ml), dried (MgSO₄), and evaporated under reduced pressure. The residue was purified by flash chromatography (SiO2, hexane-EtOAc = 3:1) to give the product (17.22 g, 93%) as oils.
(d) Preparation of (S,S)- 3,3'-Dibenzyloxy-hydrobenzoin (3a):
   A solution of dichloro(p-cymene)ruthenium (II) dimer (2.5 mg, 0.004 mmol) and (1R,2R)-N-p-toluenesulfonyl-1,2-diphenylethylenediamine (3.3 mg, 0.009 mmol) in DMF (5 ml) was stirred for 20min at room temperature, then 3,3'-dibenzyloxybenzoin (2a) (3.40 g, 8 mmol), formic acid (0.94 ml, 24.8 mmol) and Et₃N (2.90 ml, 20.8 mmol) were added. The mixture was degassed by four freeze-thaw cycles, and stirred at 40 °C for 24 h. The solvents were removed under reduced pressure. The residue was dissolved in EtOAc (20 ml), washed with water (2x10 ml) and brine (10 ml), dried (MgSO₄), and evaporated under reduced pressure. The residue was purified by flash chromatography (SiO₂, hexane-EtOAc = 3:1) to give the product (3.18 g, 93%) as oils. ¹H NMR (CDCl₃, δ): 7.31~7.39 (m, 10H), 7.14 (t, J = 7.8 Hz, 2H)), 6.84 (ddd, J = 7.8, 2.5 and 1.3 Hz, 2H), 6.79 (dd, J = 2.5 and 1.3 Hz, 2H), 6.69 (dt, J = 7.8 and 1.3 Hz, 2H), 4.95 (s, 4H), 4.64 (s, 2H), 2.85 (s, 2H).
(e) Preparation of (S,S)- 3,3'-Dibromo-hydrobenzoin (3b):
   A solution of dichloro(p-cymene)ruthenium (II) dimer (2.8 mg, 0.0045 mmol) and (1 R,2R)-N-p-toluenesulfonyl-1,2-diphenylethylenediamine (3.7 mg, 0.01 mmol) in DMF (5 ml) was stirred for 20min at room temperature, then 3,3'-dibenzyloxybenzoin (2a) (3.33 g, 9 mmol), formic acid (1.05 ml, 27.9 mmol) and Et₃N (3.26 ml, 23.4 mmol) were added. The mixture was degassed by four freeze-thaw cycles, and stirred at 40 °C for 24 h. The solvents were removed under reduced pressure. The residue was dissolved in EtOAc (20 ml), washed with water (2x10 ml) and brine (10 ml), dried (MgSO4), and evaporated under reduced pressure. The residue was purified by flash chromatography (SiO2, hexane-EtOAc = 3:1) to give the product (3.06 g, 91 %) as oils. ¹H NMR (CDCl₃, δ): 7.39 (d, J = 7.7 Hz, 2H), 7.37 (s, 2H), 7.10 (t, J = 7.7 Hz, 2H), 6.95 (d, j = 7.7 Hz, 2H), 4.62 (s, 2H), 2.96 (s, 2H).
(f) Preparation of (1S,2S)-1,2-Di(3-benzyloxyphenyl)-1,2-ditosyloxyethane (4a):
   To a solution of (S,S)- 3,3'-dibenzyloxy-hydrobenzoin (3a) (10.00 g, 23.4 mmol) in pyridine (50 ml) was added portion-wise TsCl (10.73 g, 56.30 mmol) at 0 °C. The reaction mixture was stirred at room temperature for 4 days, quenched with ice water (100 ml) and extracted with CH₂Cl₂ (3x50 ml). The combined organic layers were washed with 10% HCl (2x50 ml), saturated NaHCO₃ solution (50 ml) and brine (50 ml), dried (MgSO₄), and evaporated under reduced pressure to give the crude product, which was used directly in next step without further purification. ¹H NMR (CDCl₃, δ): 7.52 (d, J = 8.1 Hz, 4H), 7.30~7.42 (m, 10 H), 7.11 (d, J = 8.1 Hz, 4H), 6.97 (t, J = 7.8 Hz, 2H), 6.75 (dd, J = 7.8 and 2.4 Hz, 2H), 6.50 (d, J = 7.8 Hz, 2H), 5.53 (s, 2H), 4.82 (d, J = 12.7 Hz, 2H), 4.78 (d, J = 12.7 Hz, 2H), 2.33 (s, 6H). ¹³C NMR (CDCl₃, δ): 158. 80, 144.85, 137.07, 135.41, 134.07, 129.78, 129.55, 128.41, 128.29, 120.64, 116.34, 113.95, 83.71, 70.30, 21.91.
(g) Preparation of (1S,2S)-1,2-Di(3-bromophenyl)-1,2-ditosyloxyethane (4b):
   To a solution of (S,S)- 3,3'-dibromo-hydrobenzoin (3b) (8.56 g, 23.0 mmol) in pyridine (50 ml) was added portionwise TsCl (10.52 g, 55.2 mmol) at 0 °C. The reaction mixture was stirred at room temperature for 4 days, quenched with ice water (100 ml) and extracted with CH₂Cl₂ (3x50 ml). The combined organic layers were washed with 10% HCl (2x50 ml), saturated NaHCO₃ solution (50 ml) and brine (50 ml), dried (MgSO₄), and evaporated under reduced pressure to give the crude product, which was used directly in next step without further purification. ¹H NMR (CDCl₃, δ): 7.50 (d, J = 8.3 Hz, 4H), 7.30 (ddd, J = 7.8, 1.8 and 1.0 Hz, 2H), 7.16 (d, J = 8.3 Hz, 4H), 6.98~7.02 (m, 4H), 6.91 (dd, J = 7.8 and 1.0 Hz, 2H), 5.49 (s, 2H), 2.39 (s, 6H). ¹³C NMR (CDCl₃, δ): 145.39, 135.99, 133.46, 132.44, 130.73, 130.10, 130.01, 128.15, 126.49, 122.71, 82.46, 21.99.
(h) Preparation of (1R,2R)-1,2-Di(3-benzyloxyphenyl)ethane-1,2-diazide (5a):
   A suspension of the crude (1S,2S)-1,2-Di(3-benzyloxyphenyl)-1,2-ditosyloxyethane (4a) (~23 mmol) and NaN₃ (3.90 g, 60 mmol) in DMF (100 ml) was stirred at 75 °C for 8 h. After cooling to room temperature, water (300 ml) was added, and extracted with EtOAc (4x50 ml), The combined organic layers were washed with water (2x100 ml),and brine (100 ml), dried (MgSO₄), and evaporated under reduced pressure. The resulting brown oil was purified by flash chromatography (SiO₂, hexahe-EtOAc = 8:1) to give the product (6.13 g, 55%) as yellow oils. ¹H NMR (CDCl₃, δ): 7.38 (m, 10H), 7.14 (t, J = 7.7 Hz, 2H), 6.86 (dm, J = 7.7 Hz, 2H), 6.69 (m, 2H), 6.63 (d, J = 7.7 Hz, 2H), 4.96 (s, 4H), 4.55 (s, 2H).
(i) Preparation of (1R,2R)-1,2-Di(3-bromophenyl)ethane-1,2-diazide (5b):
   This compound was prepared in the same way as compound (5a). Yield: 70%. ¹H NMR (CDCl₃, δ): 7.40 (dm, J = 7.8 Hz, 2H), 7.25 (dd, J = 1.8 and 1.3 Hz, 2H), 7.13 (t, J = 7.8 Hz, 2H), 6.96 (d, J = 7.8 Hz, 2H), 4.96 (s, 4H), 4.56 (s, 2H). ¹³C NMR (CDCl₃, δ): 137.72, 132.07, 130.71, 130.14, 125.34, 122.76, 69.91.
(j) Preparation of (1R,2R)-1,2-Di(3-benzyloxyphenyl)ethane-1,2-diamine (6a):
   To a solution of (1R,2R)-1,2-di(3-benzyloxyphenyl)ethane-1,2-diazide (5a) (4.77 g, 10 mmol) in Et₂O was added portionwise LiAlH₄ (3.14 g, 83 mmol) at 0 °C. The resulting suspension was refluxed for 2 h, and then stirred at room temperature for overnight. Saturated Na₂SO₄ aqueous solution was carefully added to the reaction mixture. The solid was filtered off, and the filtrate was dried (Na₂SO₄) and evaporated under reduced pressure. The residue was triturated with hexane to give the product (4.08 g, 96%) as white solid. ¹H NMR (CDCl₃, δ): 7.29~7.43 (m, 10H), 7.19 (t, J = 7.8 Hz, 2H), 6.92 (dd, J = 2.4 and 1.8 Hz, 2H), 6.87 (br. d, J = 7.8 Hz, 2H), 6.84 (ddd, J = 7.8, 2.4 and 0.6 Hz, 2H), 5.02 (s, 4H), 4.05 (s, 2H), 1.54 (s, 4H). ¹³C NMR (CDCl₃, δ): 159.19, 145.59, 137.47, 129.62, 128.93, 128.29, 127.86, 119.96, 114.00, 113.90, 70.41, 62.15.
(k) Preparation of (1 R,2R)-1,2-Di(3-bromophenyl)ethane-1,2-diamine (6b):
   This compound was prepared in the same way as compound 6a. Yield: 97%.

### Example 2: Preparation of PEG-modified TsDPEN

Peg-modified TS DPEN was prepared according to the following scheme; 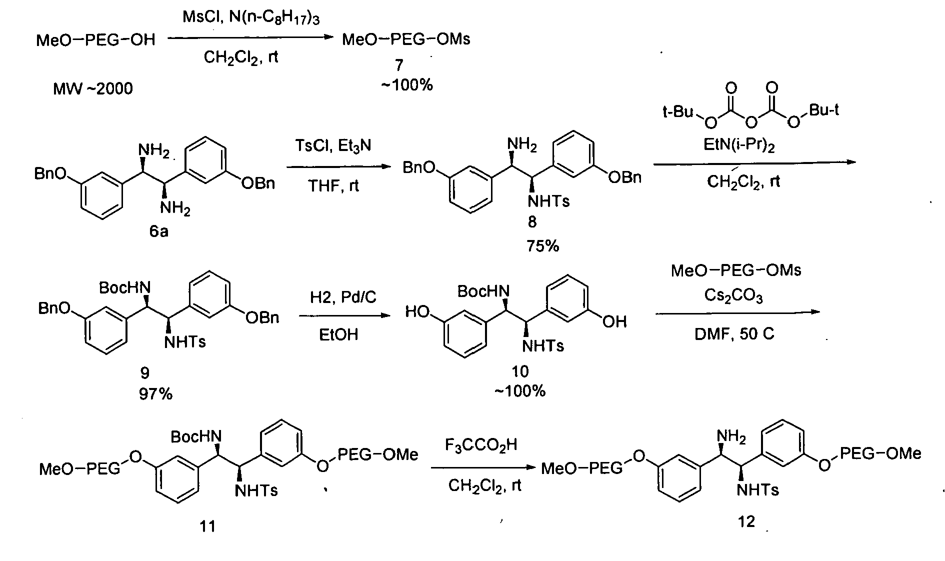
(a) Preparation of Polyethylene glycol 2000 monomethyl ether mesylate (7)
   To a solution of polyethylene glycol 2000 monomethyl ether (20.00 g, 10 mmol) and tri-n-octylamine (13.10 ml, 30 mmol) in CH₂Cl₂ (50 ml) was added MsCl (1.55 ml, 20 mmol) at 0 °C. The reaction mixture was stirred for 24 h at room temperature, and poured into Et₂O (1000 ml) with stirring. After stirring for 30 min at 0 °C, the precipitate was collected by filtration, washed with Et₂O (5x200 ml), and dried in vaccum to give the product (20.50 g) as white solid.
(b) Preparation of (R,R)-N-Tosyl-1,2-di(3-benzyloxyphenyl)ethane-1,2-diamine (8)
   To a solution of (R,R)-1,2-di(3-benzyloxyphenyl)ethane-1,2-diamine (6a) (1.27 g, 3 mmol) and pyridine (2 ml) in THF (15 ml) was added dropwise a solution of TsCl (572 mg, 3 mmol) in THF (5 ml) at 0 °C. The reaction mixture was then allowed to warm to room temperature and stirred overnight. THF was removed under reduced pressure. The residue was dissolved in CH₂Cl₂ (30 ml), washed with saturated NaHCO₃ aqueous solution (10 ml), dried (MgSO₄), and evaporated under reduced pressure. The residue was purified by flash chromatography (SiO₂, hexane-EtOAc = 1:4) to give the product (1.31 g, 75.3%) as white solid.
(c) Preparation of (R,R)-N-Boc-N'-tosyl-1,2-Di(3-benzyloxyphenyl)ethane-1,2-diamine (9)
   To a solution of (R,R)-N-Tosyl-1,2-di(3-benzyloxyphenyl)ethane-1,2-diamine (8) (579 mg, 1.0 mmol) and N,N-diisopropylethylamine (0.26 ml, 1.5 mmol) in CH₂Cl₂ (5 ml) was added di-tert-butyl dicarbonate (240 mg, 1.1 mmol). The reaction mixture was stirred overnight at room temperature, washed with 1 N HCl (5 ml) and brine (5 ml), dried (MgSO₄), and evaporated under reduced pressure. The residue was purified by flash chromatography (SiO₂, hexane-EtOAc = 4:1) to give the product (649 mg, 95.6%) as white solid.
(d) Preparation of (R,R)-N-Boc-N'-tosyl-1,2-Di(3-hydroxyphenyl)ethane-1,2-diamine (10)
   A suspension of (R,R)-N-Boc-N'-tosyl-1,2-di(3-benzyloxyphenyl)ethane-1,2-diamine (9) (600 mg, 0.88 mmol) and 10% Pd/C (20 mg) in EtOH (5 ml) was stirred for 2 h at room temperature under 10 bar of hydrogen. The reaction mixture was filtered through a pad of Celite, and the solvent was then removed under reduced pressure. The residue was purified by flash chromatography (SiO₂, hexane-EtOAc = 1:1) to give the product (462 mg, 92.6%) as white foam.
(e) PEG-modified protected diamine (11)
   A suspension of (R,R)-N-Boc-N'-tosyl-1,2-di(3-hydroxyphenyl)ethane-1,2-diamine (10) (249 mg, 0.5 mmol), polyethylene glycol 2000 monomethyl ether mesylate (7) (2.00 g, 1.0 mmol) and Cs₂CO₃ (978 mg, 3.0 mmol) in DMF (10 ml) was stirred overnight at 50 °C. DMF was removed under reduced pressure; CH₂Cl₂ (10 ml) was then added. The insoluble salts were filtered off, and the filtrate was poured into Et₂O (150 ml) with stirring. After stirring for 30 min at 0 °C, the precipitate was collected by filtration, washed with Et₂O (5x20 ml), and dried in vaccum to give the product (2.15 g) as off-white solid.
(f) PEG-modified TsDPEN (12):
   A solution of protected PEG-modified diamine (11) (2.0 g) in CH₂Cl₂ (5 ml) and CF₃CO₂H (5 ml) was stirred for 4 h at room temperature. The solvent was removed under reduced pressure. The residue was dissolved in CH₂Cl₂ (10 ml), and tri-n-octylamine (5 ml) was added. The mixture was stirred for 30 min at room temperature, and poured into Et₂O (150 ml) with stirring. After stirring for 30 min at 0 °C, the precipitate was collected by filtration, washed with Et₂O (5x20 ml), and dried in vaccum to give the product (1.93 g) as off-white solid.

### Example 3: Preparation of PEG-modified DPEN

PEG-modified DPEN was prepared according to the following scheme; 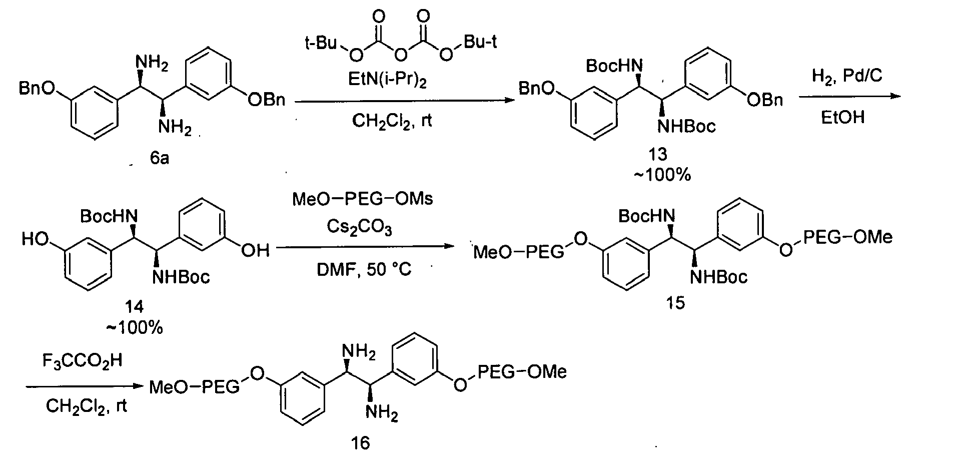
(a) Preparation of (R,R)-N,N-Bis-Boc-1,2-di(3-benzyloxyphenyl)ethane-1,2-diamine (13):
   To a solution of (R,R)-1,2-di(3-benzyloxyphenyl)ethane-1,2-diamine (6a) (1.32 g, 3.1 mmol) and N,N-diisopropylethylamine (1.21 ml, 7.0 mmol) in CH₂Cl₂ (10 ml) was added di-tert-butyl dicarbonate (1.42 g, 6.5 mmol). The reaction mixture was stirred overnight at room temperature, washed with 1 N HCl (5 ml) and brine (5 ml), dried (MgSO₄), and evaporated under reduced pressure. The residue was purified by flash chromatography (SiO₂, hexane-EtOAc = 4:1) to give the product (1.88 g, 97.3%) as white solid.
(b) Preparation of (R,R)-N,N-Bis-Boc-1,2-di(3-hydroxyphenyl)ethane-1,2-diamine (14):
   A suspension of (R,R)-N,N-Bis-Boc-1,2-di(3-benzyloxyphenyl)ethane-1,2-diamine (13) (1.50 g, 2.4 mmol) and 10% Pd-C (50 mg) in EtOAc (10 ml) was stirred for 2 h at room temperature under 10 bar of hydrogen. The reaction mixture was filtered through a pad of Celite, and the solvent was removed under reduced pressure. The residue was purified by flash chromatography (SiO₂, hexane-EtOAc = 1:1) to give the product (1.00 g, 93.7%) as white foam.
(c) PEG-modified protected diamine (15):
   A suspension of (R,R)-N,N-Bis-Boc-1,2-di(3-hydroxyphenyl)ethane-1,2-diamine (14) (222 mg, 0.5 mmol), polyethylene glycol 2000 monomethyl ether mesylate (55) (2.00 g, 1.0 mmol) and Cs₂CO₃ (978 mg, 3.0 mmol) in DMF (10 ml) was stirred overnight at 50 °C. DMF was removed under reduced pressure; CH₂Cl₂ (10 ml) was added. The insoluble salts were filtered off, and the filtrate was poured into Et₂O (150 ml) with stirring. After stirring for 30 min at 0 °C, the precipitate was collected by filtration, washed with Et₂O (5x20 ml), and dried in vacuum to give the product (2.10 g) as off-white solid.
(d) PEG-modified DPEN (16)
   A solution of PEG-modified protected diamine (15) (2.0 g) in CH₂Cl₂ (5 ml) and CF₃CO₂H (5 ml) was stirred for 4 h at room temperature. Most of solvents were removed under reduced pressure. The residue was dissolved in CH₂Cl₂ (10 ml), and tri-n-octylamine (5 ml) was added. The mixture was stirred for 30 min at room temperature, and poured into Et₂O (150 ml) with stirring. After stirring for 30 min at 0 °C, the precipitate was collected by filtration, washed with Et₂O (5x20 ml), and dried in vacuum to give the product (1.95 g) as pale-yellow solid.

### Example 4: Preparation of Insoluble polymer-supported DPEN

Polymer supported DPEN was prepared according to the following scheme; 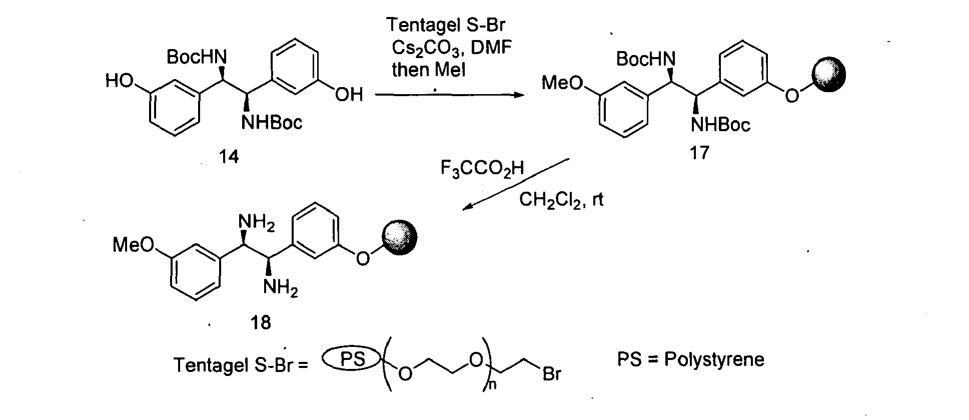
(a) Preparation of protected supported diamine (17):
   A suspension of (R,R)-N,N-Bis-Boc-1,2-di(3-hydroxyphenyl)ethane-1,2-diamine (14) (311 mg, 0.7 mmol), Tentagel S-Br (0.2~0.3 mmol/g, 2.00 g, ~0.5 mmol) and Cs₂CO₃ (978 mg, 3.0 mmol) in DMF (10 ml) was stirred 24 h at 70 °C. After cooling to room temperature, Mel (0.1 ml, 1.6 mmol) was added, and the suspension was stirred for another 16 h at 40 °C. After cooling to room temperature, the mixture was diluted with H₂O (20 ml). The solid was collected by filtration, washed with H₂O, MeOH, acetone and Et₂O, dried in vacuum to give polymer (17) (2.06 g) as yellow solid.
(b) Preparation of supported diamine (18):
   A suspension of polymer (17) (2.06 g) in CH₂Cl₂ (5 ml) and CF₃CO₂H (5 ml) was stirred overnight at room temperature. The solid was filtered off, suspended in 5% Na₂CO₃ aqueous solution (10 ml), and stirred for 4 h at room temperature. The solid was collected by filtration, washed with H₂O, MeOH, acetone and Et₂O, dried in vacuum to give polymer (18) (1.99 g) as yellow solid.

### Example 5: Asymmetric hydrogenation

### (S)-α-Methyl-1-naphthalenemethanol

Under argon, a solution of polymer-supported DPEN (18) (100 mg,) and [RuCl₂((R)-BINAP)(dmf)₂] (4.7 mg, 0.005 mmol) in DMF-i-PrOH (1:1) (5 ml) was stirred for 10 min at room temperature in an autoclave. Pre-degassed 0.1 M t-BuOK in i-PrOH (0.3 ml, 0.03 mmol) and 1-acetonaphthone (850 mg, 5 mmol) were added. Hydrogen was introduced into the autoclave at a pressure of 10 bar, then carefully released. After repeating this procedure three times, the autoclave was pressurised to 50 bar. The reaction mixture was stirred for 20 hours at room temperature. The catalyst was separated from the supernatant and re-used. The yield and ee of (S)-α-Methyl-1-naphthalenemethanol was determined by GC analysis. The results were as follows;

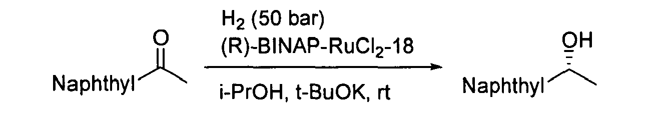

| | s/c | time (h) | Conversion (%) | ee (%) |
|---|---|---|---|---|
| Run 1 | 1000 | 20 | 97 | 86 |
| Run 2 | 1000 | 20 | 99 | 95 |

The results show the effectiveness of the catalyst upon re-use is not diminished.

## Claims

1. A ligand of formula (I) 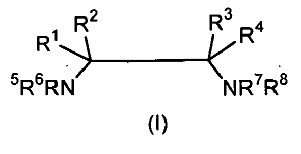 wherein R¹, R³, R⁵, R⁶, R⁷ and R⁸ are hydrogen, **characterized in that** R² and R⁴ are functional group-containing aryl groups in which the functional groups are selected from the group consisting of halogen (Cl, Br, F or I), hydroxyl, alkoxy, carbonyl, carboxyl, anhydride, carbene, methacryl, epoxide, vinyl, nitrile, mercapto, amine, imine, amide and imide.

2. A ligand according to claim 1 selected from the list consisting of; 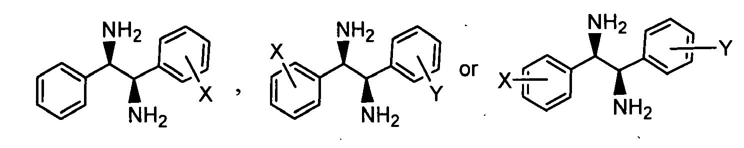 where X and Y are functional groups.

3. A ligand according to claim 1 or claim 2 consisting of a 1,2-di-(meta-substituted phenyl) ethylene diamine.

4. A ligand according to any one of claims 1 to 3 of formula; 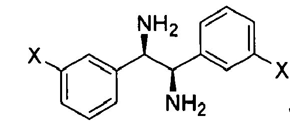 where X = Br or OBn (benzyl).

5. A method for preparing a ligand of formula (I) 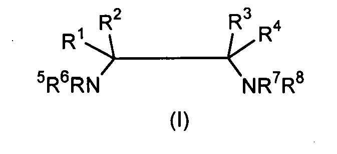 wherein R¹, R³, R⁵, R⁶, R⁷ and R⁸ are hydrogen and R² and R⁴ are functional group-containing aryl groups in which the functional groups are selected from the group consisting of halogen (Cl, Br, F or I), hydroxyl, alkoxy, carbonyl, carboxyl, anhydride, carbene, methacryl, epoxide, vinyl, nitrile, mercapto, amine, imine, amide and imide comprising the steps of ;
(a) Performing a benzoin condensation on a functionalised benzaldehyde to give a functionalised benzoin,
(b) reducing the functionalised benzoin by asymmetric hydrogen transfer hydrogenation using a Ru catalyst to give a functionalised hydrobenzoin,
(c) transforming the functionalised hydrobenzoin via a diazide into a functionalised 1,2-diarylamine.

6. A method according to claim 5 wherein the functionalized benzaldehyde is 3-bromobenzaldehyde or 3-benzyloxybenzaldehyde.
